# EUROPEAN PATENT APPLICATION

(11) **EP 1 570 794 A1**
(43) Date of publication of application: **07.09.2005**
(21) Application number: 04005056.9
(22) Date of filing: 04.03.2004
(51) Int. Cl.: A61B 17/70

(54) **Bone fixation apparatus, method and tool for assembling the same**

(71) Applicant: U & I Corporation, Kyunggi-do 480-080 (KR)
(72) Inventor: Jeon, Chang-Hun, Seoul 137-030 (KR)
(74) Representative: Flaccus, Rolf-Dieter

(57) **Abstract**

Disclosed is a bone fixation apparatus. This apparatus comprises a bone screw having a head; a cap member placed on the head of the bone screw; a receiver member having a bore in which the cap member and the head of the bone screw are respectively accommodated and held, and a U-shaped channel through which a support bar extends; and a compression member threadedly coupled into the receiver member to downwardly bias the support bar; wherein a multitude of stepped portions are formed at the lower end and on the inner surface of the receiver member, an accommodating chamber is defined above the stepped portions to accommodate therein the cap member, internal threads are formed on the inner surface of the receiver member to allow the compression member to be threadedly coupled therewith, and an outward flange is formed around the upper end of the cap member, thereby preventing movement of a bone, improving the assemblability of the bone fixation apparatus and keeping a cap member from being released.

## Description

### FIELD OF THE INVENTION

The present invention relates to a bone fixation apparatus which is used for fixing and stabilizing a bone such as the spine, and so forth, after the bone is corrected into its normal state.

### BACKGROUND OF THE INVENTION

Various bone fixation apparatuses have been disclosed in the art, such as those described in Korean Patent Laid-open Publication No. 2000-48562 and U.S. Patent No. 6,280,442.

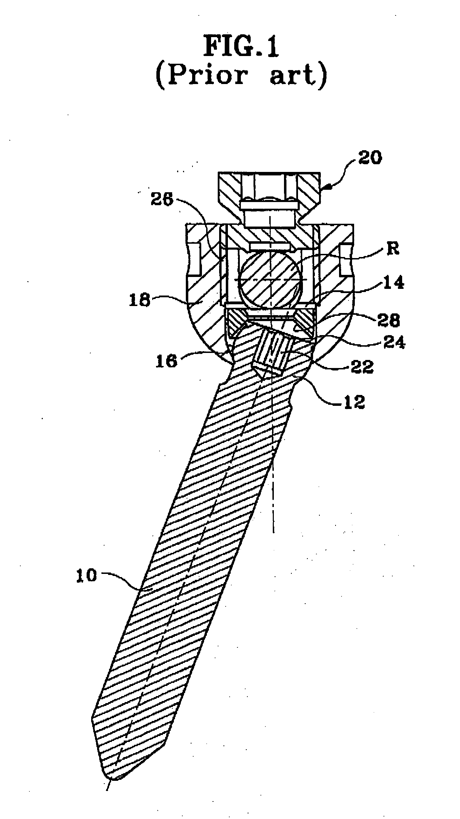

As shown in FIG. 1, the bone fixation apparatus described in Korean Patent Laid-open Publication No. 2000-48562 includes a bone screw 10, a shrinkage collet 14, a receiver member 18, and a set screw 20. The bone screw 10 has a spherical head 12. The shrinkage collet 14 functions to support the head 12 of the bone screw 10. The receiver member 18 has a center bore for delimiting a tapered recess 16 in which the shrinkage collet 14 is accommodated. The receiver member 18 further has a U-shaped channel that communicates with the recess 16 and through which a support bar R extends. The set screw 20 is threadedly coupled to the receiver member 18 to downwardly bias the support bar R.

The head 12 of the bone screw 10 is defined with a tool-engaging groove 22 in which a tool can be engaged. The tool engaging groove 22 is defined on a flat upper end surface 24 of the head 12, in which the upper end surface 24 is formed by truncating the head 12. A portion of the receiver member 18 which defines the U-shaped channel is formed with internal threads 26 so that the set screw 20 can be threadedly coupled to the internal threads 26. The lower surface of the shrinkage collet 14 is formed to have a contoured depression 28 in which the head 12 is partially accommodated. In the contoured depression 28, the lower part of the shrinkage collet 14 is formed with a plurality of slots, so that a desired pressing force is applied to the head 12 of the bone screw 10.

If the set screw 20 is tightened, the support bar R compresses the shrinkage collet 14, and the shrinkage collet 14 is squeezed within the tapered recess 16 of the receiver member 18 in such a way as to fixedly hold the bone screw 10 at a vertical or inclined position.

Meanwhile, as shown in FIG. 2, in the bone fixation apparatus described in U.S. Patent No. 6,280,442, a series of ridges 34 are formed on the head 32 of a bone screw 30, a retainer ring 38 is fitted adjacent to the lower end of a receiver member 36 so that the head 32 can be retained by the retainer ring 38. A cap member 40 is placed on the upper part of the head 32.. Above the cap member 40, a support bar R is inserted through a U-shaped channel defined in the receiver member 36 and then biased downward by a compression member 42.

In the receiver member 36, the lower part in which the cap member 40 is inserted is formed to have an inner diameter greater than that of the upper part into which the compression member 42 is threadedly coupled. Due to this fact, even when the compression member 42 is unscrewed and the support bar R is removed, the cap member 40 is prevented from being released in an upward direction. When assembling the bone fixation apparatus, after the cap member 40 and the head 32 of the bone screw 30 are sequentially inserted through the lower end of the receiver member 36, the retainer ring 38 is placed around and moved upward on the bone screw 30 and then fitted into an inward annular groove defined adjacent to the lower end of the receiver member 36.

The internal threads of the receiver member 36 may be formed in a manner such that the cap member 40 is also threadedly coupled to the internal threads to be prevented from being released from the receiver member 36.

However, in the former bone fixation apparatus as described in Korean Patent Laid-open Publication No. 2000-48562, the head 12 supported in the tapered recess 16 is likely to be moved by an external factor because the supporting force is insufficient. Consequently, the head 12 cannot be reliably maintained in an initially supported state.

The bone fixation apparatus as described in U.S. Patent No. 6,280,442, while coping to some extent the problems caused in Korean Patent Laid-open Publication No. 2000-48562, suffers from defects in that it is difficult to fit the retainer ring 38 in place, and the supporting force of the bone screw is still insufficient.

When the head of the bone screw is supported by the cap member threadedly coupled to the receiver member, it is not easy to screw the cap member adjacent to the lower end of the receiver member. Further, because the biasing force of the compression member cannot be transferred to the head, assemblability is deteriorated and the supporting force of the bone screw is downgraded.

### SUMMARY OF THE INVENTION

The present invention provides a bone fixation apparatus with an improved supporting force of a bone screw, thereby preventing movement of a bone, improving the assemblability of the bone fixation apparatus and keeping a cap member from being released.

According to an embodiment of the present invention, there is provided a bone fixation apparatus comprising: a bone screw having a head; a cap member placed on an upper part of the head of the bone screw; a receiver member having a bore in which the cap member and the head of the bone screw are respectively accommodated and held, and a U-shaped channel through which a support bar extends; and a compression member threadedly coupled into the receiver member to downwardly bias the support bar; wherein a multitude of stepped portions are formed at a lower end of and on an inner surface of the receiver member to be brought into linear contact with an outer surface of a lower part of the head so that the supporting force for the head of the bone screw is increased, an accommodating chamber is defined above the stepped portions to accommodate therein the cap member, and internal threads having an inner diameter smaller than that of the accommodating chamber are formed on an inner surface of the receiver member and threadedly coupled with the compression member; and wherein an outward flange having an outer diameter larger than the inner diameter of the internal threads is formed around an upper end of the cap member in a manner such that the outward flange of the cap member is plastically deformed along with a lower end of the internal threads while being accommodated in the accommodating chamber.

According to another embodiment of the present invention, a plurality of grooves are defined on an outer surface of the head of the bone screw in a manner such that they are spaced apart from each other in such a way to increase frictional force between the head and the cap member; and the plurality of grooves are asymmetrically defined with respect to an axis of the bone screw in a manner such that an inner edge of a lower end of the cap member extends across some of the grooves irrespective of the assembling angle of the bone screw. The annular groove has a depth of 0.01~0.03 mm.

According to another embodiment of the present invention, the receiver member is configured in a manner such that the middle portion of the receiver member through which the support bar extends has a diameter larger than the upper and lower portions of the receiver member.

According to another embodiment of the present invention, there is provided a method for assembling the bone fixation apparatus, comprising the steps of:
slantingly inserting the cap member into the accommodating chamber in a manner such that one side of the outward flange is accommodated in the accommodating chamber and the opposite side of the outward flange leans against the lower end of the internal threads; and pressing the cap member downward using a compressing tool so that the opposite side of the outward flange is inserted into the accommodating chamber while being plastically deformed on the lower end of the internal threads.

According to still another embodiment of the present invention, there is provided an assembling tool for setting up the bone fixation apparatus, comprising: an alignment rod having a lower end projection which is fitted into a wrench insertion groove of the bone screw, and external threads which are to be threadedly coupled with the internal threads of the receiver member to align the bone screw and the receiver member of the bone fixation apparatus with each other; and a handle coupled to the alignment rod to rotate the alignment rod.

According to yet still another embodiment of the present invention, the assembling tool further comprises an outer pipe having a pair of leg portions which, are inserted into the U-shaped channels of the receiver member so that the threading and unthreading force for the bone screw is increased and withdrawal of the receiver member is eased, the outer pipe being fitted around the alignment rod in a manner so that the outer pipe can slidably receive the alignment rod in an axial direction. It is preferred that a grip is formed on the end of the outer pipe to allow easy manipulation of the outer pipe.

### BRIEF DESCRIPTION OF THE DRAWINGS

The above objects, and other features and advantages of the present invention will become more apparent after a reading of the following detailed description when taken in conjunction with the drawings, in which:
FIG. 1 is a cross-sectional view illustrating a conventional bone fixation apparatus;

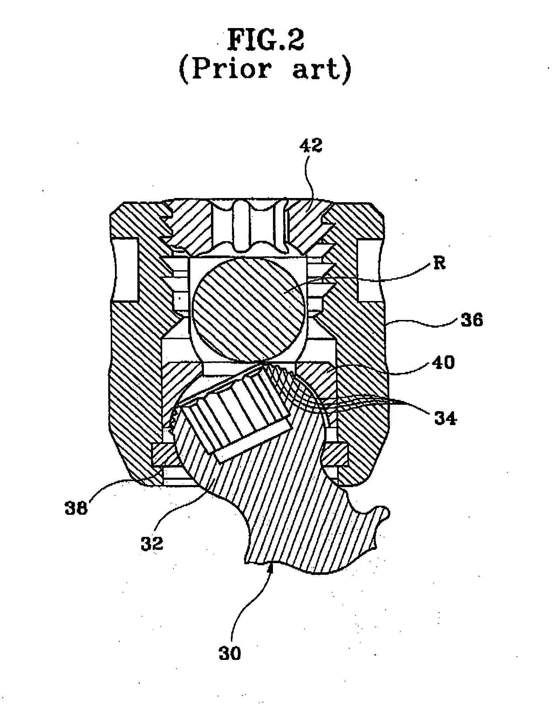

FIG. 2 is a cross-sectional view illustrating another conventional bone fixation apparatus;
FIG. 3 is a cross-sectional view illustrating a bone fixation apparatus in accordance with an embodiment of the present invention;
FIG. 4 is an exploded perspective view illustrating the bone fixation apparatus according to the present invention;
FIG. 5 is a cross-sectional view illustrating a receiver member of FIG. 3;
FIG. 6 is a cross-sectional view illustrating a cap member of FIG. 3;
FIG. 7 is a partially enlarged front view illustrating a head of a bone screw shown in FIG. 3;
FIG. 8 is a cross-sectional view illustrating a state wherein the cap member of the bone fixation apparatus according to the present invention is assembled in place;
FIG. 9 is a perspective view illustrating an in-use status of the bone fixation apparatus according to the present invention;
FIG. 10 is an exploded perspective view illustrating a tool used for assembling the bone fixation apparatus according to the present invention;
FIG. 11 is a cross-sectional view illustrating an assembling position of the tool shown in FIG. 10;
FIG. 12 is a graph reflecting the results of tests conducted in respect of the present bone fixation apparatus and the conventional bone fixation apparatuses; and
FIG. 13 is a schematic view for explaining a condition under which each bone fixation apparatus is tested.

### DETAILED DESCRIPTION OF PREFERRED EMBODIMENTS

Reference will now be made in greater detail to a preferred embodiment of the invention, an example of which is illustrated in the accompanying drawings. Wherever possible, the same reference numerals will be used throughout the drawings and the description to refer to identical or like parts.

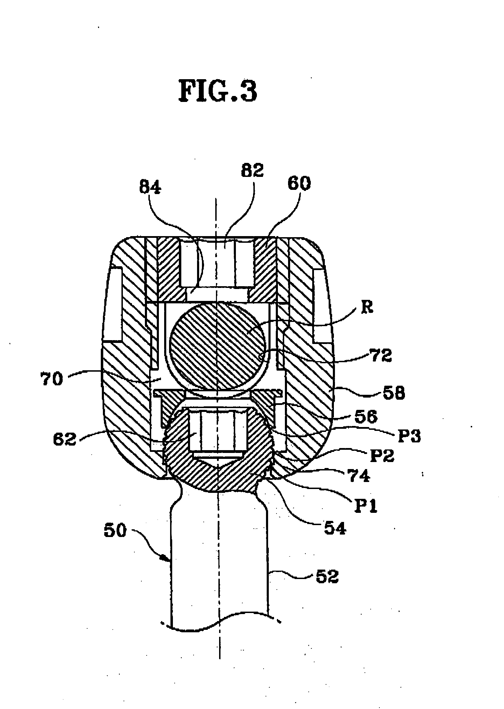

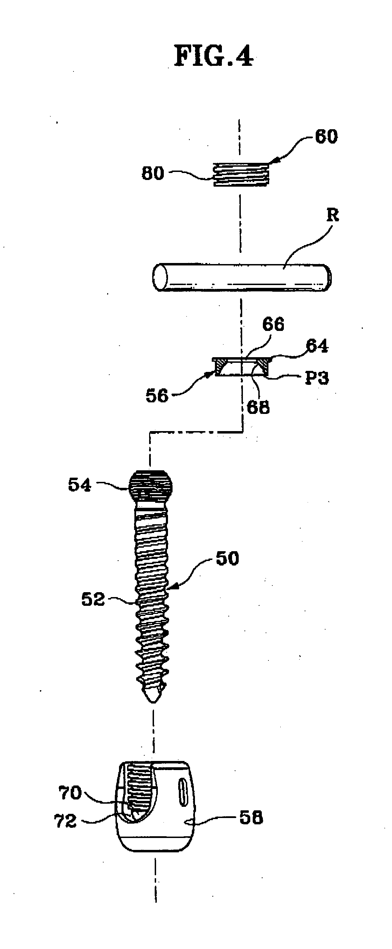

As shown in FIGs. 3 and 4, a bone fixation apparatus in accordance with an embodiment of the present invention includes a bone screw 50 which has external threads 52 and a head 54. The upper part of the head 54 of the bone screw 52 is supported by a cap member 56. The bone fixation apparatus further includes a receiver member 58 in which the head 54, the cap member 56 and a support bar R are accommodated and fixedly held. A compression member 60 for fixing the support bar R is threadedly coupled into the upper part of the receiver member 58.

The external threads 52 of the bone screw 50 are screwed into a bone. The lower end of the external threads 52 is pointed to be easily screwed into the bone. Although the root diameter of the external threads 52 is gradually decreased toward the lower end, the same crest diameter of the external threads 52 is maintained. If the external threads of a bone screw have the same outer diameter and the same thread height throughout the entire length of the bone screw, a problem is caused in that, the bone screw is apt to shake because the fixing force is gradually decreased due to a repetitive application of external force, etc. In an effort to cope with this problem, while a structure is disclosed in the art in which an outer diameter is gradually decreased toward the lower end of the bone screw, this structure encounters a drawback in that, due to the reduction in strength, the bone screw is likely to be broken. In consideration of this problem and drawback, in the present invention, the root diameter of the external threads is gradually decreased toward the lower end of the bone screw and the external threads have the same crest diameter. As a consequence, not only are the external threads securely fixed with respect to the bone, it is also possible to prevent the strength of the bone screw from being reduced.

The head 54 of the bone screw 50 has a: truncated sphere-shaped configuration. A hexagonal groove 62 of a certain depth is defined on the flat upper end surface of the head 54 so that a tool such as a wrench and the like can be inserted into the hexagonal groove 62 to screw the bone screw 50 into the bone. It is to be noted that the head may have a groove of various sectional shapes.

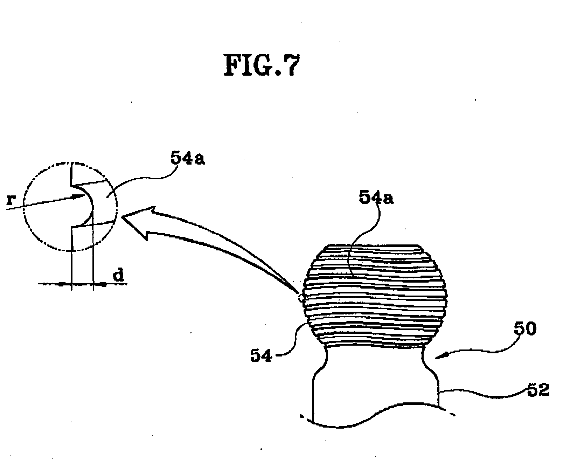

Further, as shown in FIG. 7, a plurality of annular grooves 54a are defined on the outer surface of the head 54 of the bone screw 50 in a manner so that they are spaced apart from each other by a predetermined distance. The annular grooves 54a serve to increase frictional force between the head 54 and the cap member 56. The annular grooves 54a are asymmetrically defined with respect to the axis of the bone screw 50 in a manner so that the inner edge of the lower end of the cap member 56 extends across some of the annular grooves 54a irrespective of the assembling angle of the bone screw 50. For example, the annular grooves 54a may be defined to constitute a spiral groove. Each annular groove 54a has a depth d of 0.01~0.03 mm and a radius r of about 0.2 mm, and is preferred to be continuously formed in order to minimize debris, burr and metal chips and the like from being generated by linear contact with the cap member when the bone fixation apparatus is assembled, performed or removed.

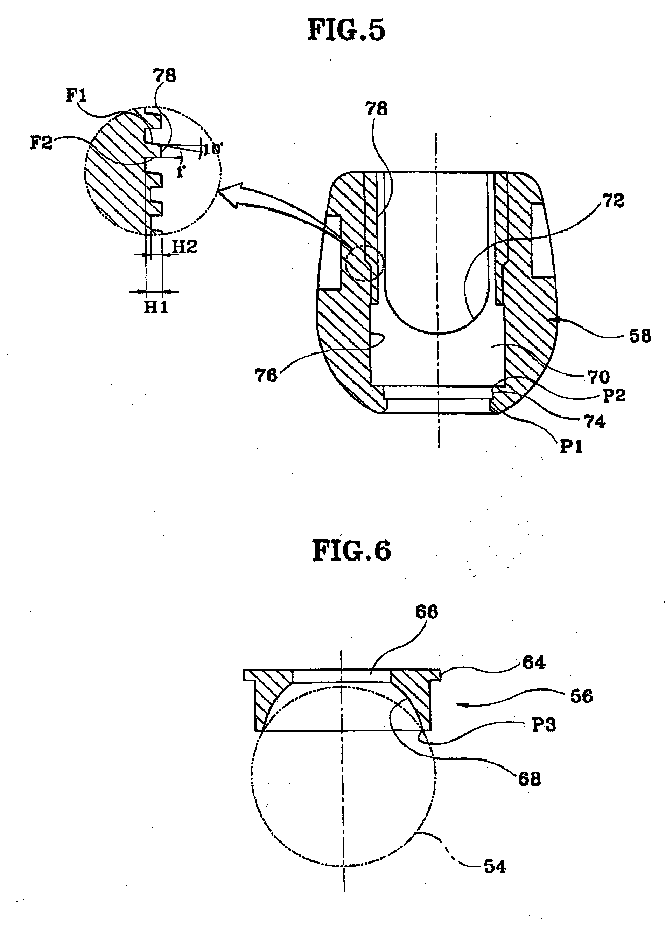

As can be readily seen in FIG. 6, the cap member 56 substantially has a disk-shaped configuration with an outward flange 64 formed on the upper end thereof. The outward flange 64 is formed to have an outer diameter larger than the inner diameter of the internal threads of the receiver member 58. A hole 66 is defined through the center portion of the cap member 56 to communicate with the hexagonal groove 62. A recess 68 of a certain depth is defined on the lower surface of the cap member 56. The recess 68 possesses a rounded surface which has a radius of a curvature less than that of the head 54. At a lower end P3 of the recess 68, the cap member 56 is brought into linear contact with an outer surface of the upper part of the head 54. It is to be noted that the recess 68 may have a truncated cone-shaped contour.

As shown in FIG. 5, the receiver member 58 has a bore 70 which extends in an axial direction and in which the cap member 56 and the head 54 of the bone screw 50 are respectively accommodated and fixedly held. The receiver member 58 further has a U-shaped channel 72 through which the support bar R extends. The receiver member 58 is configured in a manner such that the middle portion thereof through which the support bar R extends has a diameter larger than the upper and lower portions thereof.

While the largest inertial moment is developed in a region where the support bar R extends through the receiver member 58, the receiver member 58 has an increased strength and is prevented from being distorted due to the fact that the receiver member 58 is configured with the middle portion of the receiver member 58 having a diameter larger than the upper and lower portions thereof. Furthermore, in most cases, the support bar R is bent relative to the anatomical structure of the spine when operation is performed. When the support bar R is bent, an interference (or collision) may occur among the two or more fixation apparatuses coupled to a support bar R. At this time, the interference largely occurs among the receiver members which are the most bulky components of the bone fixation apparatus, where the interference is minimized due to the configuration of the receiver member. Also, an interference may be generated between the spine and the fixation apparatus, and the interference between the spine and the fixation apparatus becomes minimal when the lower end of the receiver member is configured with the middle portion thereof having a larger diameter than the upper and lower portions thereof.

Two stepped portions 74 are formed at a lower end and inner surface of the receiver member 58 to increase the supporting force for the head 54. Edges P1 and P2 of the stepped portions 74 are brought into linear contact with the outer surface of the lower part of the head 54. It is to be readily understood that three or four stepped portions 74 may be formed in place of the two stepped portions 74. The upper edge P2 of an upper stepped portion 74 has an inner diameter greater than that of the lower edge P1 of the lower stepped portion 74, in which the edges P1 and P2 of the stepped portions 74 can be brought into linear contact with the outer surface of the lower part of the spherical head 54.

The bore 70 is formed therein with stepped portions 74, an accommodating chamber 76 defined above the stepped portions 74 to accommodate the cap member 56, and internal threads 78 to which the compression member 60 is threadedly coupled.

The accommodating chamber 76 has an inner diameter greater than that of the internal threads 78. As the cap member 56 is assembled according to an assembling method of the present invention, as will be described later in detail, the outward flange 64 of the cap member 56 undergoes plastic deformation along with the lower end of the internal threads 78, in a manner such that the cap member 56 is prevented from being unintentionally and upwardly released from the receiver member 58 after being inserted into the accommodating chamber 76.

The upper portion of the internal threads 78 has a thread height H1 greater than a thread height H2. of the lower portion of the internal threads 78, so that the compression member 60 can be threadedly moved only on the upper portion and cannot be threadedly moved on the lower portion of the internal threads 78. Therefore, the compression member 60 is screwed only up to such a depth to downwardly bias the support bar R. In this regard, due to the fact that the compression member 60 can be positioned in a proper place even when the support bar R is not inserted through the U-shaped channel 72, assembly of the bone fixation apparatus can be easily performed within a short period of time.

A person skilled in the art will readily recognize that the internal threads 78 may have the same thread height and define the same contour at the upper and lower portions thereof.

The internal threads 78 comprise trapezoidal threads, so that deformation of the receiver member 58 can be prevented and the internal threads 78 can provide optimal results in terms of force transfer, locking efficiency, and likelihood of unscrewing. In this regard, in the case of a triangular thread, as the compression member 60 is screwed, while excellent locking force can be obtained, the axial force transfer rate becomes low. Also, because the receiver member 56 widens in a radial direction after locking of the compression member 60, a disadvantage is caused in that a separate cap member must be coupled to the receiver member 58. Further, a square thread suffers from defects in that, while an excellent axial force transfer rate is obtained, the likelihood of unscrewing is increased. In the present invention, due to the fact that trapezoidal threads are adopted, optimal results are provided in terms of force transfer and locking efficiency, and it is not necessary to couple a separate cap member to the receiver member.

It is preferred that the trapezoidal thread has an upper surface which is formed to have a slope F1 greater than a slope F2 of a lower surface. Thus, the compression member 60 can be easily assembled while being prevented from being unintentionally unscrewed. Concretely speaking, the upper and lower surfaces of the trapezoidal thread have slopes F1 and F2 of 10° and 1°, respectively. Namely, since the upper slope is greater than the lower slope, the compression member 60 can be easily screwed downward. Also, deformation is minimized even when force is upwardly applied. Moreover, since the engagement between the internal threads 78 and the compression member 60 is maximized, precision is improved and unintentional unscrewing of the compression member 60 is avoided.

The compression member 60 has a cylinder-shaped configuration with threads 80 formed on the circumferential outer surface thereof. The threads 80 are threadedly coupled to the internal threads 78 of the receiver member 58. A hexagonal groove 82 and a hole 84 which are communicated with each other are defined at the center portion of the compression member 60.

Hereafter, the assembly of the bone fixation apparatus according to the present invention, constructed as mentioned above, will be described.

First, in the state where the bone screw 50 is positioned above the receiver member 58, the bone screw 50 is inserted downward into the bore 70 of the receiver member 58, in a manner such that the head 54 is seated on the multiple stepped portions 74. Then, the cap member 56 is moved downward through the internal threads 78 to be positioned in the accommodating chamber 76.

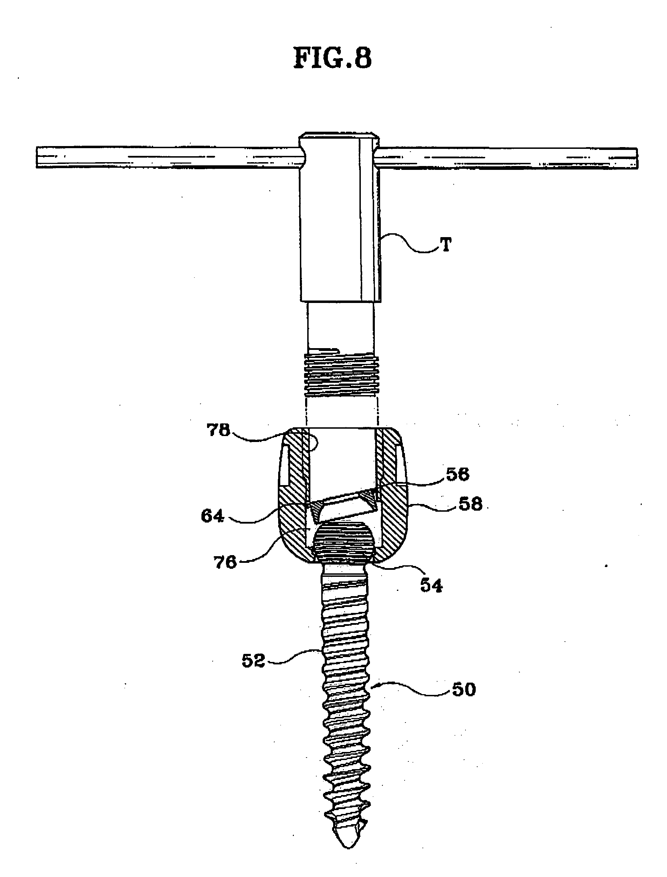

When assembling the cap member 56, as shown in FIG. 8, the cap member 56 is slantingly inserted into the accommodating chamber 76 in a manner such that only one side of the outward flange 64 is accommodated in the accommodating chamber 76 and the opposite side of the outward flange 64 leans against the lower end of the internal threads 78. Thereafter, by pressing the cap member 56 downward using a compressing tool T, the opposite side of the outward flange 64 is plastically deformed along with the lower end of the internal threads 78. As the cap member 56 undergoes plastic deformation, the cap member 56 accommodated in the accommodating chamber 76 is prevented from being released upward.

A tool which is formed on the lower portion thereof with external threads to be coupled with the internal threads 78 is used as the compressing tool T. At this time, an outer diameter of the external threads of the compressing tool T is determined in consideration of the thread height H2 of the lower portion of the internal threads 78 in a manner such that the external threads can engage completely through the internal threads 78.

Then, the assembling tool, as will be described later in detail, is threadedly moved downward through the receiver member 58 in a manner such that its lower end is fitted into the hexagonal groove 62 of the head 54 after passing through the hole 66 of the cap member 56. After the receiver member 58 and the bone screw 50 are aligned with each other in this way, the assembling tool is rotated to drive the bone screw 50 into the bone. Thereupon, the support bar R is inserted through the U-shaped channel 72 to be placed over the cap member 56, and the compression member 60 is screwed into the receiver member 58 to fix the bone screw 50.

The locking force of the compression member 60 is transferred through the support bar R and the cap member 56 to the head 54. At this time, due to the fact that the edges P1 and P2 of the stepped portions 74 of the receiver member 58 are brought into linear contact with the outer surface of the lower part of the head 54 and at the lower end P3 of the recess 68, the cap member 56 is brought into linear contact with the outer surface of the upper part of the head 54, and the bone screw 50 is securely fixed against movement.

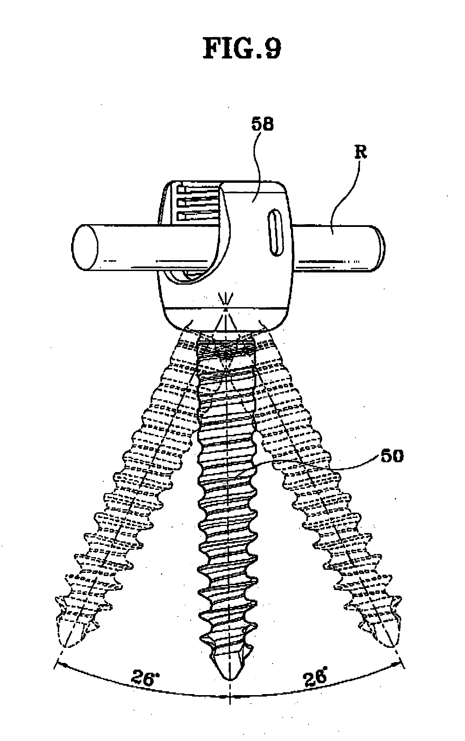

As shown in FIG. 9, the bone screw 50 can be fixed so that it is inclined within 26° when measured from the vertical axis. At this time, due to the fact that the plurality of annular grooves 54a constituting a spiral groove are asymmetrically defined on the outer surface of the head 54 of the bone screw 50, the lower end P3 of the recess 68 of the cap member 56 extends across some of the annular grooves 54a irrespective of the assembling angle of the bone screw 50. Consequently, the frictional force can be increased between the head 54 and the cap member 56.

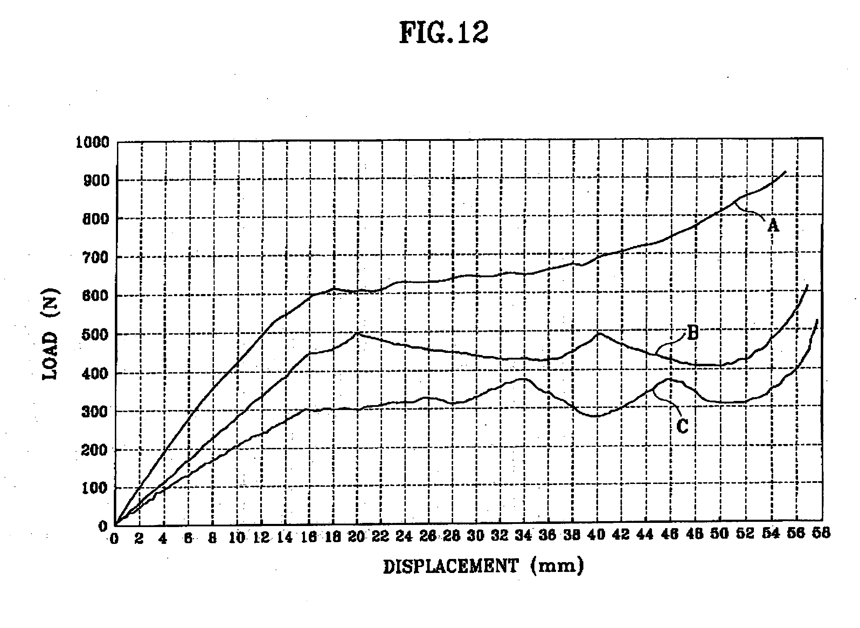

FIG. 12 is a graph obtained by testing the present bone fixation apparatus and the conventional bone fixation apparatuses in terms of the supporting force of bone screws. In each test, a compression member is screwed into a receiver member with a locking torque of 14 Nm, and a displacement is measured when a head is yielded under static load application.

A universal compression tester having Model No. MTS 793 was used as the test equipment, and each test was implemented in accordance with ASTM F1717.

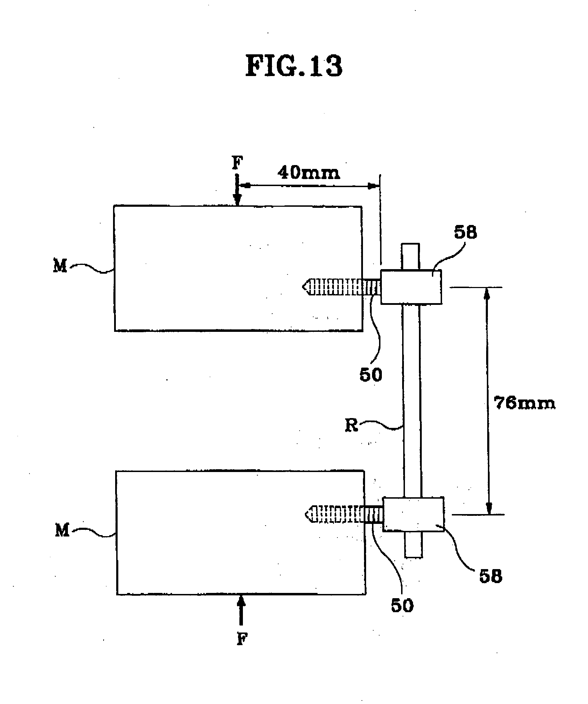

FIG. 13 is a schematic view for explaining the conditions under which each bone fixation apparatus is tested. As shown in FIG. 9, bone screws 50 are driven into the upper and lower objects M to have a spacing of 76 mm. Thereafter, the support bar R is inserted through the receiver members 58 of the bone screws 50, and the compression members (not shown) are screwed into the receiver members 58. Then, by applying a load F to each bone screw at a location 40 mm from the head of each bone screw, deformation of the corresponding object was observed to obtain the graph as shown in FIG. 8.

In the graph, line A represents the bone fixation apparatus manufactured according to the present invention, line B represents the conventional bone fixation apparatus manufactured according to U.S. Patent No. 6,280,442, and line C represents the conventional bone fixation apparatus manufactured according to Korean Patent Laid-open Publication No. 2000-48562.

As can be readily seen from the graph, while the bone fixation apparatus according to the present invention has a yielding point of 550 N, the conventional bone fixation apparatuses respectively have yielding points of 450 N and 300 N. Therefore, it is to be readily understood that the bone fixation apparatus according to the present invention provides increased supporting force.

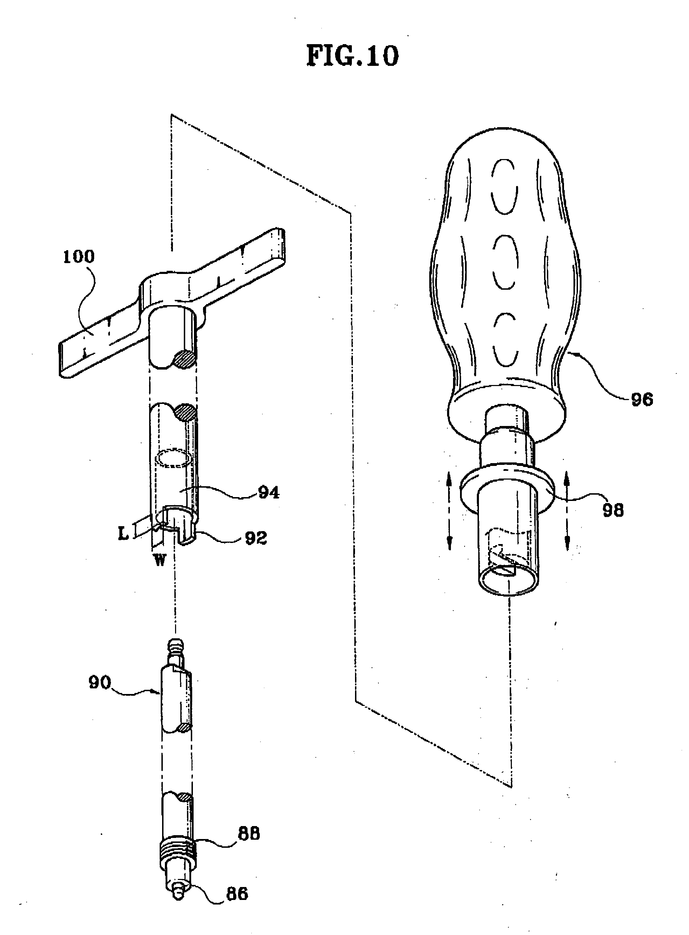

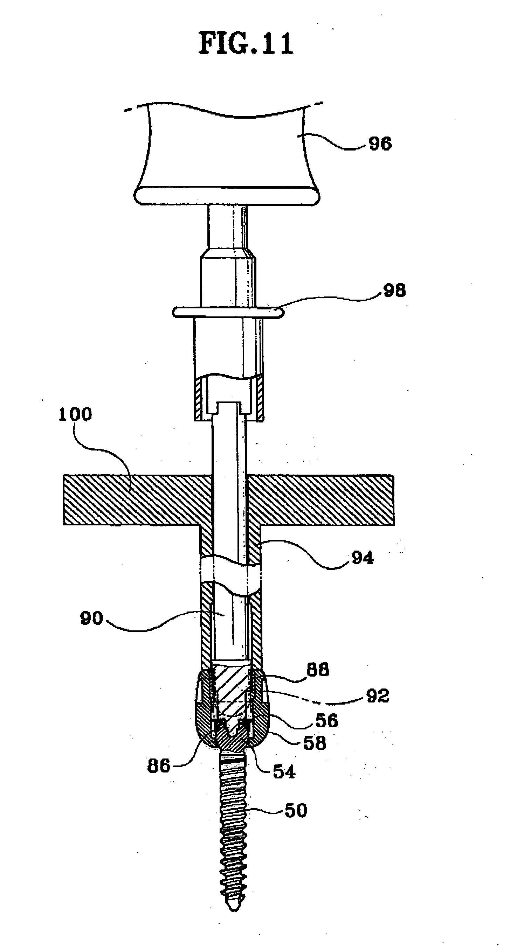

FIGs. 10 and 11 illustrate the assembling tool that is used when fixing the bone screw 50 into the bone with the head 54 accommodated in the receiver member 58.

As shown in the drawings, the assembling tool that is used to assemble the bone fixation apparatus according to the present invention includes an alignment rod 90, an outer pipe 94, and a handle 96. The alignment rod 90 has a lower end projection 86 which is fitted into the hexagonal groove 62 of the bone screw 50 which serves as a wrench insertion groove, and external threads 88 which are to be threadedly coupled with the internal threads 78 of the receiver member 58. The alignment rod 90 functions to align the bone screw 50 and the receiver member 58 of the bone fixation apparatus with each other. The outer pipe 94 has a pair of leg portions 92 which are to be inserted into the U-shaped channels 72 of the receiver member 58 so that the threading and unthreading force for the bone screw 50 is increased and withdrawal of the receiver member 58 is eased. The outer pipe 94 is fitted around the alignment rod 90 in a manner such that the outer pipe 94 can slidably receive the alignment rod 90 in an axial direction. The handle 96 is coupled to the alignment rod 90 to rotate the alignment rod 90.

While it can be envisaged that the handle 96 is integrally coupled to the alignment rod 90, it is preferred that, as shown in FIG. 10, the alignment rod 90 is detachably coupled to the end of the alignment rod 90 by a conventional coupling means. A movable switching pipe 98 is connected to the handle 96 in a manner such that it can be moved by the elastic force of a spring to lock and unlock the alignment rod 90.

A grip 100 is formed on the upper end of the outer pipe 94. The grip 100 extends in a direction perpendicular to the axis of the outer pipe 94 to easily move the outer pipe 94. A width W and a length L of the leg portion 92 are determined on the basis of the dimensions of the U-shaped channel 72.

In the assembling tool structured as described above, as can be readily seen in FIG. 11, if the handle 96 is rotated with the external threads 88 of the alignment rod 90 initially coupled with the internal threads 78 of the receiver member 58, the lower end projection 86 of the alignment rod 90 is fitted into the hexagonal groove 62 of the bone screw 50, by which the receiver member 58 and the bone screw 50 are aligned with each other along a straight line. Then, by continuously rotating the handle 96, the bone screw 50 can be easily driven into the bone.

At this time, by inserting the pair of leg portions 92 of the outer pipe 94 into the U-shaped channels 72 of the receiver member 58 and then simultaneously rotating the handle 96 and the grip 100 of the outer pipe 94, the threading force of the bone screw 50 into the bone is increased to ease the bone fixing task. Further, since the receiver member 58 is engaged with the leg portions 92, when unthreading the bone screw 50, the receiver member 58 can also be easily removed.

As apparent from the above description, the bone fixation apparatus according to the present invention provides advantages in that the supporting force of a bone screw is increased to prevent movement of a bone and the assemblability of the bone fixation apparatus is improved. Further, in the bone fixation apparatus according to the present invention, the force transmission rate is increased, and an unintentional unscrewing of a compression member is avoided.

In the drawings and specification, there have been disclosed typical preferred embodiments of the invention, and although specific terms are employed, they are used in a generic and descriptive sense only and not for purposes of limitation, the scope of the invention being set forth in the following claims.

## Claims

1. A bone fixation apparatus comprising:
a bone screw having a head;
a cap member placed on the upper part of said head of said bone screw;
a receiver member having a bore in which said cap member and said head of said bone screw are respectively accommodated and held, and a U-shaped channel through which a support bar extends; and
a compression member threadedly coupled into said receiver member to downwardly bias said support bar;
wherein a multitude of stepped portions are formed at the lower end and on the inner surface of said receiver member to be brought into linear contact with the outer surface of the lower part of said head so that the supporting force for said head of said bone screw is increased, an accommodating chamber is defined above said stepped portions to accommodate therein said cap member, and internal threads having an inner diameter smaller than that of said accommodating chamber are formed on the inner surface of said receiver member and threadedly coupled with said compression member; and
wherein an outward flange having an outer diameter larger than the inner diameter of said internal threads is formed around the upper end of said cap member in a manner such that said outward flange of said cap member is plastically deformed along with the lower end of said internal threads while being accommodated in said accommodating chamber.

2. The bone fixation apparatus as set forth in claim 1, wherein
a plurality of grooves are defined on the outer surface of said head of said bone screw in a manner such that they are spaced apart from each other in such a way as to increase frictional force between said head and said cap member; and
said plurality of grooves are asymmetrically defined with respect to the axis of said bone screw in a manner such that the inner edge of the lower end of said cap member extends across some of said plurality of grooves irrespective of the assembling angle of said bone screw.

3. The bone fixation apparatus as set forth in claim 2, wherein said plurality of grooves have a depth of 0.01∼0.03 mm.

4. The bone fixation apparatus as set forth in claim 1, wherein said receiver member is configured in a manner such that the middle portion of said receiver member through which the support bar extends has a diameter larger than the upper and lower portions of said receiver member.

5. A method for assembling the bone fixation apparatus as set forth in claim 1, comprising the steps of:
slantingly inserting said cap member into said accommodating chamber in a manner such that one side of said outward flange is accommodated in said accommodating chamber and the opposite side of said outward flange leans against the lower end of said internal threads; and
pressing said cap member downward using a compressing tool so that the opposite side of said outward flange is inserted into said accommodating chamber while being plastically deformed on the lower end of said internal threads.

6. An assembling tool for setting up the bone fixation apparatus as set forth in claim 1, comprising:
an alignment rod having a lower end projection which is fitted into a wrench insertion groove of said bone screw, and external threads which are to be threadedly coupled with said internal threads of said receiver member to align said bone screw and said receiver member of the bone fixation apparatus with each other; and
a handle coupled to said alignment rod to rotate said alignment rod.

7. The assembling tool as set forth in claim 6, further comprising:
an outer pipe having a pair of leg portions which are to be inserted into said U-shaped channel of said receiver member so that the threading and unthreading force for said bone screw is increased and withdrawal of said receiver member is eased, said outer pipe being fitted around said alignment rod in a manner such that said outer pipe can slidably receive said alignment rod in an axial direction.
